# EUROPÄISCHE PATENTANMELDUNG

(11) **EP 3 398 912 A1**
(43) Veröffentlichungstag der Anmeldung: **07.11.2018**
(21) Anmeldenummer: 17169714.7
(22) Anmeldetag: 05.05.2017
(51) Int. Cl.: C02F 11/04, C12M 1/107, C12M 1/00, C02F 3/28, C02F 1/36, C02F 1/44, C02F 3/34, C02F 1/78, C02F 1/38

(54) **VERFAHREN UND VORRICHTUNG ZUR LEISTUNGSSTEIGERUNG ANAEROBER ABBAUVERFAHREN DURCH ERWEITERUNG BZW. ANPASSUNG DER VORVERSÄUERUNGSSTUFE**

(71) Anmelder: Hochschule für Angewandte Wissenschaften Hof, 95028 Hof (DE)
(72) Erfinder: Schmid, Andreas, 95703 Schönkirch (DE)
(74) Vertreter: Engelhard, Markus

(57) **Zusammenfassung**

Die vorliegende Erfindung bezieht sich auf eine Vorrichtung zur Vorversäuerung, ein Verfahren zur Vorversäuerung und die Verwendungen der Vorrichtung und des Verfahrens, bei der Herstellung von Biogas oder anderen anaeroben Abbauprodukten auf Basis organischer Ausgangssubstrate oder in der Abwasser- und Schlammbehandlung.

## Beschreibung

Die vorliegende Erfindung bezieht sich auf eine Vorrichtung zur Vorversäuerung, ein Verfahren zur Vorversäuerung und die Verwendungen der Vorrichtung und des Verfahrens, bei der Herstellung von Biogas oder anderen anaeroben Abbauprodukten auf Basis organischer Ausgangssubstrate oder in der Abwasser- und Schlammbehandlung.

### Hintergrund der Erfindung

Anaerobe Reaktoren, insbesondere zur anaeroben Abwasserreinigung, lassen sich nach der Art des Biomasserückhalts und nach der Art der Biomasse unterscheiden. Diese kann flockig oder granulär vorliegen oder auf Trägermaterialien angesiedelt sein. Weiterhin lassen sich anaerobe Reaktoren nach der Raumabbauleistung unterscheiden, wobei derzeit die EGSB (expanded granular sludge blanket)- und Fließbettreaktoren die höchsten Raumabbauleistungen auch im praktischen Betrieb nachgewiesen haben [1, 5]. In Deutschland wurden bis zum Dezember 2008 ca. 250 großtechnische Anaerobanlagen zur Industrieabwasserbehandlung errichtet [6, 7]. Wichtige Kenngrößen für die Reaktorauslegung sind das Schlammalter und die Schlammbelastung, da nicht der Reaktor, sondern die im Reaktor befindliche Biomasse für die Abbauleistung verantwortlich ist. Der Biomassengehalt ist wiederum eine Funktion des Biomassenrückhalts im System, so dass zur Vereinfachung der Betrachtung die CSB (Chemischer Sauerstoffbedarf)-Raumbelastung herangezogen wird. Wesentlicher Bestandteil der anaeroben Anlagen ist die Hydrolyse-Stufe (auch Vorversäuerung genannt), die auch dann wichtig ist, wenn bereits ein hoher Gehalt an flüchtigen Säuren im Abwasser vorliegt. Diese Stufe kann getrennt angelegt sein (ggf. auch in Kombination mit einem hydraulischen Puffer) oder - abhängig vom gewählten Verfahren - in den Anaerobreaktor integriert werden. Derzeit erfolgt eine pH-Anpassung und gegebenenfalls Stickstoff- und Phosphordosierung nach dem Vorversäuerungsreaktor unmittelbar im Zustrom des Anaerobreaktors [1].

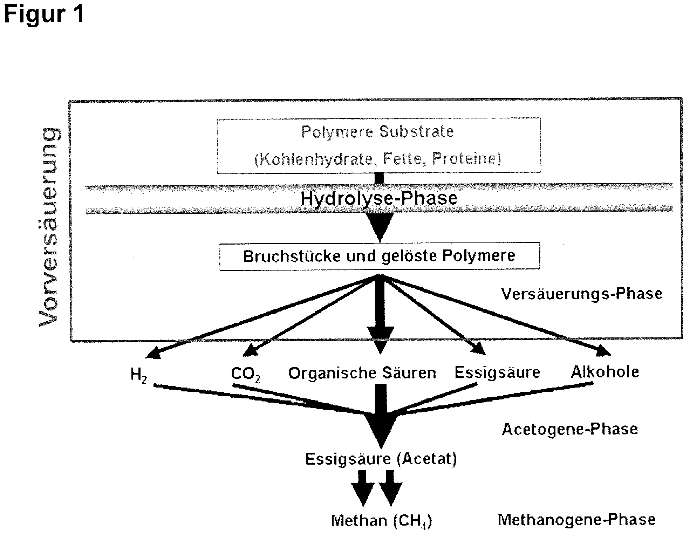

Zum Ausgleich von Mengen-, Konzentrations- und Frachtschwankungen haben sich Misch- und Ausgleichsbecken bewährt. Diese Anlagen können gleichzeitig zur gezielten Versäuerung genutzt werden, wobei die Prozesse des Ausgleichs und der Versäuerung schwer zu entkoppeln sind. Durch die vorgeschaltete Vorversäuerung wird der 4-stufige anaerobe Abbau auf zwei Reaktoren aufgeteilt (siehe Figur 1), was i.d.R. die nachfolgende Aceto- und Methanogenese stabilisiert und die Gefahr einer Übersäuerung drastisch vermindert [1].

Abhängig von der Abwasserart werden in der industriellen Abwasserreinigung für Ausgleich und Versäuerung Aufenthaltszeiten von 6 bis 24 Stunden gewählt. Bei Untersuchungen von Weiland und Wulfert [5, 6] mit Kartoffelschlempe kam es bei Überschreitung der Verweilzeit von 24 Stunden zur Bildung von Buttersäure, Valeriansäure und Capronsäure. Bei Versuchen mit anderen agrarindustriellen Abläufen nahm darüber hinaus die Konzentration von Propionsäure zu. Bei den meisten Untersuchungen zeigte sich, dass eine Verweilzeit von ca. 24 Stunden aus technisch-ökonomischen Gründen das Optimum darstellt. Einige Abwässer (zum Beispiel Kaffeeproduktionsabwasser) weisen allerdings auch bei niedrigeren Aufenthaltszeiten von 6 h noch einen hohen Versäuerungsgrad von 80 % auf [7].

Der optimale Versäuerungsgrad (Verhältnis von CSB der organischen Säuren zum filtrierten CSB) ist system- und abwasserspezifisch. Von Pelletschlammreaktoren ist bekannt, dass der Versäuerungsgrad Einfluss auf die Pelletstruktur hat. Ein vollständig versäuertes Abwasser führt nach [8] zum Zerfall der Pellets. Eine stabile Versäuerung von Schlempe aus der Zitronensäureproduktion erreichte Moser [9] bei Aufenthaltszeiten von ca. 2-3 Tagen durch Rückführung von Schlamm aus dem Methanreaktor (EKJ-Reaktor) in den Versäuerungsreaktor.

Derzeit finden sich Vorversäuerungssysteme vorwiegend bei anaeroben Hochschlastreaktoren und werden in der kommunalen Schlammbehandlung als auch in der landwirtschaftlichen Biogastechnik nicht angewendet [10].

WO 2009/155587 A2 beschreibt eine Vorrichtung beziehungsweise ein Verfahren zur Erzeugung von Methangas, bei dem mit Hilfe einer Anode, Kathode und methanogenen Mikroorganismen ausgestatteten Reaktors Methan erzeugt wird. Eine Vorversäuerung etc. wird nicht beschrieben.

Die Gewinnung von Biogas aus nachwachsenden Rohstoffen mit anschließender Verstromung wird im Erneuerbare Energie Gesetz (EEG) 2014 stark eingeschränkt. Die Folgen für die Biogasbranche sind dramatisch. Beim Anlagenneubau droht ein völliges Aus, bei den bestehenden Anlagen zumindest ein weitgehender Innovationsstopp. Die über Jahrzehnte gerade auch in Bayern erarbeitete Kompetenz in diesem Bereich droht mit den entsprechenden Firmen verloren zu gehen. Landwirten, Genossenschaften und Kommunen, die sich im Bereich Biogas engagiert haben, fehlt die Perspektive. Dabei könnte Biogas, als originär stofflicher Energieträger, wichtige Funktionen im Kontext der Energiewende erfüllen (Treibstoff, Energiespeicher, Regelenergieträger).

Dazu müsste in diesem Bereich allerdings ein verfahrens- und prozesstechnisches Umdenken stattfinden. Die Anlagen der Zukunft müssen deutlich "intelligenter" und vor allem flexibler werden, sowohl was ihre "Produkte" und deren Verwendung als auch was ihre "Rohstoffbasis" betrifft. Gleichzeitig muss die Biogasproduktion in ein Gesamtkonzept Abfall/Energie integriert werden. Während dies ab einer gewissen Anlagengröße (> 30'000 t/a) bereits machbar ist, gibt es für kleine (landwirtschaftliche) bis mittlere (kommunale) Anlagen außerhalb der Großstädte, bislang keine wirtschaftlich tragbaren technischen Lösungen.

Es besteht ein Bedarf im Stand der Technik nach verbesserten Mitteln und Methoden in der Schlammbehandlung und landwirtschaftlichen Biogasnutzung.

### Vorrichtung zur Vorversäuerung

Die Aufgabe wird erfindungsgemäß durch die Bereitstellung einer Vorrichtung zur Vorversäuerung gelöst.

In einem Aspekt betrifft die Erfindung eine Vorrichtung zur Vorversäuerung von organischem Substrat, insbesondere zur Verwendung im Rahmen eines nachgeschalteten anaeroben Abbauverfahrens, wobei die Vorrichtung umfasst:
(a) einen Reaktor ("Vorversäuerungsreaktor"), umfassend
   (i) ein vorgeschaltetes Zerkleinerungsaggregat, für organisches Substrat und/oder Cosubstrat(e),
   (ii) einen Zulauf für die Zuführung von Substrat und/oder Cosubstrat, bevorzugt besagtem organischen Substrat und/oder Cosubstrat;
   (iii) jeweils einen Abfluss und Zufluss zum Verbinden des Reaktors im Umlauf mit mindestens einem Desintegrations-System;
   (iv) einen Ablauf, bevorzugt zum Verbinden des Reaktors mit einem anaeroben Reaktor;
   (v) Rührmittel, wie Rührwerk oder einer internen oder externen Umwälzpumpe;
   wobei die Vorrichtung weiterhin umfasst:
(b) mindestens einen Desintegrations-System, das sich mit dem Reaktor (a) in Umlaufverbindung befindet; und/oder
(c) einen internen Schlammkreislauf für die Anreicherung von hydrolytischen und acidophilen Mikroorganismen; und/oder
(d) eine Zugabevorrichtung zur Dosierung von Phosphor und/oder Stickstoff in den Reaktor. In einer Ausführungsform ist der interne Schlammkreislauf ein sich innerhalb des Reaktors befindender Schlammkreislauf. In einer Ausführungsform umfasst die Vorrichtung außer dem Reaktor a) jedes von b) und d).

In einer Ausführungsform umfasst die Vorrichtung zwei oder mehrere Desintegrations-Systeme (b),
und/oder wobei das oder die Desintegrations-Systeme (b) Mittel zur Durchführung eines Kavitationsverfahrens, Mittel zur Durchführung einer Elektroporation, eine Rührwerkskugelmühle, einen Scherspalthomogenisator, Mittel zur Durchführung eines Ultraschallverfahrens, Mittel zur Durchführung eines Elektroimpulsverfahrens, eine Lysat-Zentrifuge, Mittel zur Enzymzugabe, Mittel zur Durchführung von Autolyse, Mittel zur Durchführung von Nassoxidation, Mittel zur Ozondosierung, Mittel zur Durchführung von alkalischer Hydrolyse oder Mittel zur Durchführung von saurer Hydrolyse, oder Kombinationen davon umfasst (umfassen).

In einer Ausführungsform steht (stehen) das oder die Integrations-Systeme (b) mit dem Reaktor (a) im Bypass-Rücklaufstrom in Umlaufverbindung stehen.

In einer Ausführungsform umfasst der interne Schlammkreislauf (c):
Mittel zur Sedimentation im Reaktor, wie etwa einen Schrägklärer vor dem Ablauf, eine separate Sedimentationsstufe, und/oder Mittel zur internen und/oder externen Filtration.

In einer Ausführungsform umfasst die Vorrichtung weiterhin:
- mindestens ein Rückhaltesystem für die Anreicherung von hydrolytischen und/oder acidophilen Mikroorganismen und/oder für die Anreicherung von Feststoffen, zum Beispiel eine Abscheideeinrichtung oder Abscheidemittel zum Zurückhalten von Feststoffen und/oder von hydrolytischen und acidophilen Mikroorganismen, und/oder von Feststoffen, insbesondere ein (oder mehrere) Rückhaltesystem(e) ausgewählt aus:
   Mitteln zur Filtration innerhalb oder außerhalb des Reaktors, insbesondere Mittel zur internen oder externen Crossflow-Filtration, beispielsweise Mikro- oder Ultrafiltration, wobei, bevorzugt, die Mittel zur Filtration in Nassaufstellung oder Trockenaufstellung vorgesehen sind; Mittel zur Feststoffrückhaltung, insbesondere mittels Zentrifugation; Mittel zur Sedimentation in einem dem Reaktor nachgeschalteten Becken, beispielsweise einem Zwischenklärbecken; Mittel zur Sedimentation im Reaktor, bevorzugt in einer oder mehreren strömungsberuhigten Zonen des Reaktors; und Kombinationen davon;
   und/oder Mittel zur pH-Wert-Anpassung im Reaktor (a) oder im Ablauf des Reaktors.

### Verfahren zur Vorbehandlung

In einem weiteren Aspekt betrifft die Erfindung ein Verfahren zur Vorbehandlung, insbesondere Vorversäuerung, von organischem Substrat, umfassend die Schritte:
- Bereitstellen eines zu behandelnden organischen Substrats in einer Vorversäuerungsvorrichtung umfassend einen Reaktor, insbesondere einer Vorrichtung gemäß der vorliegenden Erfindung,
- Durchführen einer Hydrolyse und Acidogenese des organischen Substrats mit Hilfe von hydrolytischen und/oder acidophilen Mikroorganismen,
   wobei im Rahmen der Hydrolyse/Acidogenese des organischen Substrats
   (i) mindestens ein im Umlauf mit dem Reaktor verbundenes/betriebenes Desintegrations-Verfahren durchgeführt; und/oder
   (ii) ein innerhalb des Reaktors befindlicher Schlammkreislauf für hydrolytische und/oder acidophile Mikroorganismen betrieben; und/oder
   (iii) Stickstoff und/oder Phosphor, nach Bedarf der Mikroorganismen, in den Reaktor eindosiert; und/oder
   (iv) optional, eine pH-Wert-Anpassung durchgeführt
wird.

In einer Ausführungsform werden zwei oder mehrere Desintegrations-Verfahren (i) durchgeführt werden, und/oder wobei das oder die Desintegrations-Verfahren (i) Kavitationsverfahren, Elektroporation, Mahlen mit einer Rührwerkskugelmühle, Homogenisieren mittels eines Scherspalthomogenisators, Ultraschallverfahren, Elektroimpulsverfahren, Zentrifugation mittels einer Lysatzentrifuge, Aufschluss mittels Enzymzugabe, Autolyse, Nassoxidation, Ozondosierung, alkalische Hydrolyse, saure Hydrolyse, und/oder Kombinationen davon umfasst (umfassen).

In einer Ausführungsform umfasst das Verfahren weiterhin eine Anreicherung der hydrolytischen und/oder acidophilen Mikroorganismen, bevorzugt durch Sedimentation und/oder Filtration, insbesondere während oder nach der Hydrolyse des organischen Substrats.

In einer Ausführungsform umfasst das Verfahren weiterhin
eine Vorbehandlung des organischen Substrats und/oder von Co-Substraten, beispielsweise Biomüll oder nachwachsenden Rohstoffe,
bevorzugt umfassend eine mechanische Zerkleinerung des organischen Substrats und/oder der Co-Substrate.

In einer Ausführungsform umfasst das Verfahren weiterhin das Zurückhalten von Feststoffen, optional kombiniert mit einem Zurückhalten von hydrolytischen und/oder acidophilen Mikroorganismen zum Aufbau eines internen Schlammkreislaufs.

### Verwendung

In einem weiteren Verfahren betrifft die Erfindung eine Verwendung der Vorrichtung und/oder des Verfahrens gemäß der vorliegenden Erfindung,
- in der Biogasherstellung, insbesondere der landwirtschaftlichen Biogasherstellung,
- zur Klärschlammbehandlung,
- in der industriellen Abwasserreinigung,
- in der kommunalen Abwasserbehandlung,
- in der kommunalen Schlammbehandlung,
- zur Behandlung von organischen Abfällen,
- zur Behandlung von nachwachsenden Rohstoffen,
- zur Behandlung von Schlämmen und/oder Abwässern aus der Nahrungsmittelindustrie, chemischen Industrie, oder pharmazeutischen Industrie, und/oder
- zur kombinierten Behandlung oben genannter Einsatzstoffe, insbesondere von Klärschlamm, industriellem Abwasser, kommunalem Abwasser, kommunalem Schlamm, organischen Abfällen, nachwachsenden Rohstoffen, Schlämmen und/oder Abwässern aus der Nahrungsmittelindustrie, chemischen Industrie und/oder pharmazeutischen Industrie, als Co-Substratbeigaben.

### Verfahren zum anaeroben Abbau

In einem weiteren Aspekt betrifft die vorliegende Erfindung ein Verfahren zum anaeroben Abbau von organischem Substrat, umfassend:
a) Durchführung eines Verfahrens zur Vorversäuerung gemäß der vorliegenden Erfindung, bevorzugt in einer Vorrichtung gemäß der vorliegenden Erfindung, mit einem organischen Substrat;
b) Anaerober Abbau des (der) aus (a) resultierenden Produkts (Produkte) des Vorversäuerungsverfahrens zu diversen organischen Zwischen- und Endprodukten wie z.B. Acetat, Formiat, Propionat, Butyrat, Valerianat, Ethanol, Propanol, Butanol, Wasserstoff, weiteren organischen Säuren oder Alkoholen, und/oder Methan, bevorzugt mittels Acetogenese und/oder Methanisierung,
wobei a) räumlich und/oder zeitlich getrennt von b) durchgeführt wird.

Der Begriff "Vorrichtung zur Vorversäuerung", wie hierin verwendet, bezeichnet eine Kombination eines Reaktors mit mindestens einem Desintegrations-System und einem internen Schlammkreislauf. Der Begriff "Reaktor", wie hierin verwendet, bezeichnet einen Reaktor innerhalb einer erfindungsgemäßen Vorrichtung zur Vorversäuerung ("Vorversäuerungsvorrichtung"); er kann deshalb auch als "Vorversäuerungsreaktor" im engeren Sinne bezeichnet werden. Bei Einsatz einer erfindungsgemäßen Vorversäuerungsvorrichtung bzw. eines erfindungsgemäßen Vorversäuerungsreaktors im Rahmen eines anaeroben Abbauverfahrens, findet in der Vorversäuerungsvorrichtung bzw. dem Vorversäuerungsreaktor eine Hydrolyse und Versäuerung statt, in deren Rahmen aus organischen Substraten/Co-Substraten, bspw. organischen polymeren Substraten zunächst hydrolysierte Bruchstücke und gelöste organische Verbindungen und hieraus dann kurzkettige organische Säuren, Alkohole, CO₂ und H₂ entstehen. Die im Rahmen eines anaeroben Abbauverfahrens sich anschließende Bildung von Essigsäure, bzw. Methan (in der so genannten acetogenen Phase, bzw. methanogenen Phase) findet nicht in der erfindungsgemäßen Vorversäuerungsvorrichtung bzw. dem erfindungsgemäßen Vorversäuerungsreaktor statt. Hierdurch wird eine zeitliche und/oder räumliche Trennung der Vorversäuerungsphase, bestehend aus Hydrolyse und Versäuerung von der sich anschließenden Essigsäure/Methanproduktion erzielt. Die in der erfindungsgemäßen Vorversäuerungsvorrichtung durchgeführte Vorversäuerung kann optimiert und entsprechend den jeweiligen Bedürfnissen angepasst werden. In einem Vorversäuerungsreaktor bzw. einer Vorversäuerungsvorrichtung gemäß der vorliegenden Erfindung findet deshalb bevorzugt ausschließlich eine Hydrolyse und/oder Versäuerung, jedoch keine gezielte acetogene Phase und/oder methanogene Phase ("Methanisierung") statt. Der Begriff "Anaerobreaktor", oder anaerober Reaktor, wie hierin verwendet, bezeichnet einen Reaktor, in dem bevorzugt eine acetogene Phase bzw. eine methanogene Phase stattfindet, nicht jedoch eine Hydrolyse und/oder Versäuerung. Die beiden Vorgänge "Hydrolyse" und "Versäuerung", wie hierin verwendet, können auch gemeinsam als "Vorversäuerungsphase" bezeichnet werden. Der Begriff "intern" und "extern", wie hierin verwendet, bspw. im Kontext eines Reaktors oder einer Vorrichtung, bspw. einer Vorversäuerungsvorrichtung, bezieht sich darauf, dass der genannte Bestandteil sich innerhalb (oder außerhalb) des Reaktors bzw. der Vorrichtung befindet. Bspw. befindet sich ein "interner Schlammkreislauf" als Bestandteil einer Vorrichtung innerhalb dieser Vorrichtung. Ein solcher "interner Schlammkreislauf" kann sich jedoch auch innerhalb des Reaktors, der ja Bestandteil der Vorrichtung ist, befinden.

In einer Vorversäuerungsvorrichtung gemäß der vorliegenden Erfindung, insbesondere innerhalb eines Vorversäuerungsreaktors gemäß der vorliegenden Erfindung kann ein Rückhaltesystem oder können mehrere Rückhaltesysteme vorgesehen sein. Ein solches Rückhaltesystem kann für die Anreicherung von hydrolytischen und/oder acidophilen Mikroorganismen zum Aufbau eines internen Schlammkreislaufs eingesetzt werden. Ein solches Rückhaltesystem kann auch oder alleine für die Anreicherung von Feststoffen vorgesehen sein. In einer Ausführungsform ist ein Rückhaltesystem vorgesehen, das sowohl für die Anreicherung von hydrolytischen und acidophilen Mikroorganismen als auch für die Anreicherung von Feststoffen vorgesehen ist. In einer anderen Ausführungsform der vorliegenden Erfindung ist ein Rückhaltesystem ausschließlich für die Anreicherung von hydrolytischen und/oder acidophilen Mikroorganismen vorgesehen und ein weiteres Rückhaltesystem für die Anreicherung von Feststoffen. Ein Beispiel für ein erfindungsgemäßes Rückhaltesystem ist eine Abscheideeinrichtung oder ein Abscheidemittel zum Abscheiden bzw. Zurückhalten von Feststoffen und/oder hydrolytischen und/oder acidophilen Mikroorganismen. Weitere Beispiele von Rückhaltesystemen in Übereinstimmung mit Ausführungsformen der vorliegenden Erfindung sind Mittel zur Filtration innerhalb oder außerhalb des Reaktors oder Vorrichtung, insbesondere Mittel zur internen oder externen Cross-Flow-Filtration. Beispiele hierfür sind die Mikrofiltration oder die Ultrafiltration. Die Mittel zur Filtration können in Nassaufstellung oder Trockenaufstellung vorgesehen sein. Weitere Beispiele für Rückhaltesysteme gemäß der vorliegenden Erfindung sind Mittel zur Feststoffrückhaltung mittels Zentrifugation. Ein typisches Beispiel hierfür ist eine Zentrifuge, die Bestandteil der Vorrichtung sein kann, oder bei Bedarf hinzugezogen werden kann. Eine entsprechende Zentrifuge kann auch in die Vorrichtung integriert sein. Weitere Beispiele für Rückhaltesysteme gemäß der vorliegenden Erfindung sind Mittel zur Sedimentation in einem nachgeschalteten Becken. Ein solches Becken kann dem Reaktor gemäß der vorliegenden Erfindung nachgeschaltet sein. Ein solches Becken kann beispielsweise ein Zwischenklärbecken sein, welches nach Durchführung der Vorversäuerung dazu dient, Feststoffe und/oder Mikroorganismen zu sedimentieren und den geklärten Überstand dann anschließend zur weiteren Verarbeitung im Rahmen des weiteren anaeroben Abbaus (bspw. im Rahmen einer Acetogenese und/oder Methanogenese) in einem separaten Reaktor, dem Anaerobenreaktor, verfügbar zu machen. Weitere Beispiele für Rückhaltesystem gemäß der vorliegenden Erfindung sind beispielsweise Mittel zur Sedimentation innerhalb des Reaktors in strömungsberuhigten Zonen und/oder in eingebauten Parallelplattenabscheider / Schrägklärer. Der Begriff "Biomasse" oder "Substrat", wie hierin verwendet, bezeichnet jegliche organische Masse aus tierischen und/oder pflanzlichen Quellen. In einer Ausführungsform bezeichnet der Begriff pflanzliche Bestandteile, tierische Bestandteile, pflanzliche Abfälle, tierische Abfälle, Abwasser, nachwachsende Rohstoffe, kommunale Schlämme, industrielle Schlämme und/oder Gülle. Ein Substrat, das gemäß der vorliegenden Erfindung verarbeitet werden kann, ist bevorzugt einer oder mehrere der vorgenannten Bestandteile. In einer bevorzugten Ausführungsform sind die im Rahmen der vorliegenden Erfindung verwendeten Biomassenbestandteile Substrate insbesondere pflanzlichen Ursprungs. Beispiel für pflanzliche Substrate sind Holz, Naturfasern, Pflanzenöle, Zucker und Stärke aus Pflanzen, usw. Von ebenfalls besonderer Bedeutung für den anaeroben Abbau sind tierische Abfälle, insbesondere Gülle. Der Begriff "Biomasse" wird hierin manchmal auch als "Substrat" oder "organisches Substrat" bezeichnet. Ein solches Substrat oder organisches Substrat ist typischerweise biologisch abbaubar. Organisches Substrat, welches biologisch abbaubar ist, ist Ausgangspunkt der vorliegenden Erfindung und wird mittels der erfindungsgemäßen Vorrichtung gemäß dem erfindergemäßen Verfahren behandelt, insbesondere vorbehandelt, d.h. vorversäuert.

Ebenfalls von Bedeutung sind kommunale oder industrielle Abwässer, organische Abfälle, insbesondere Biomüll aus Haushalten und Klärschlämme aus Kommune und Industrie. Die genannten Bestandteile können auch als "Cosubstrate" eingesetzt werden, in der Gestalt, dass in der Vorrichtung gemäß der vorliegenden Erfindung neben einem Hauptbestandteil (Substrat), beispielsweise Pflanzenabfall, auch ein weiterer Bestandteil (als Cosubstrat) eingesetzt wird, beispielsweise Biomüll.

### Weitere Beschreibung von Ausführungsformen des erfindungsgemäßen Verfahrens und der erfindungsgemäßen Vorrichtung

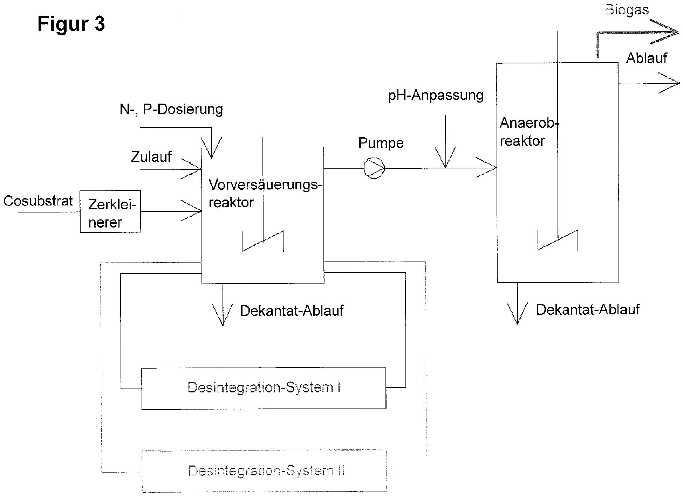

Figur 3 zeigt eine *Ausführungsform einer* erfindungsgemäßen Vorrichtung zur Vorversäuerung und das entsprechende Verfahren.

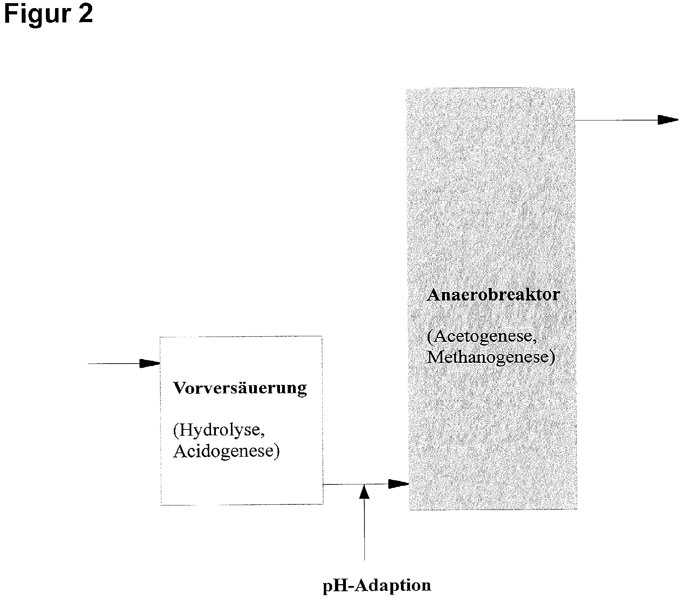

Zur Verbesserung der Betriebsstabilität wird vor dem eigentlichen Anaerobreaktor ein Vorversäuerungsreaktor vorgeschaltet (siehe Figur 2), was die Gefahr einer möglichen Übersäuerung im Anaerobreaktor durch eine pH-Wert-Adaption drastisch vermindert. Diese Betriebsweise wird derzeit nur in der industriellen Abwasserreinigung verwendet.

Für die Verwendung alternativer Cosubstrate (z.B. Biomüll) können diese Stoffe vor Eintrag in den Vorversäuerungsreaktor mechanisch zerkleinert werden. Etwaig eingetragene Feststoffe sollen durch eine Abscheideeinrichtung im Vorversäuerungsreaktor zurück gehalten werden (z.B. Einbau eines Parallelplattenabscheiders).

Durch großzügige Dimensionierung wird in einer Ausführungsform ein eigener Schlammkreislauf der hydrolytischen und acidogenen Mikroorganismen aufgebaut, was die Effizienz zusätzlich begünstigt. Ein solcher interner Schlammkreislauf wird derzeit technisch nicht in anderen Verfahren/Vorrichtungen zur Vorversäuerung angewendet.

Darüber hinaus erlaubt in einer Ausführungsform eine Dosierung von Stickstoff und Phosphor mittels einer Zugabevorrichtung die Adaption eines optimalen C:N:P-Verhältnisses zur Leistungssteigerung der hydrolytischen und acidophilen Mikroorganismen im Vorversäuerungsreaktor. Eine solche Dosierung wird derzeit technisch nicht in anderen Verfahren/Vorrichtungen zur Vorversäuerung angewendet.

Um die Hydrolyse dieser alternativen Substrate als auch die der verwendeten Schlämme zu beschleunigen, werden in einer Ausführungsform ein oder verschiedene/mehrere Desintegrationsverfahren in der Vorversäuerung im Bypass - Rücklaufstrom/Umlaufstrom betrieben; was derzeit im Stand der Technik in anderen Verfahren/Vorrichtungen nicht angewendet wird.

Die vorgenannten Ausführungsformen können alternativ oder in beliebigen Kombinationen vorgesehen sein. In einer bevorzugten Ausführungsform sind sowohl Schlammkreislauf als auch Desintegrationssystem als auch Zugabevorrichtung in einer erfindungsgemäßen Vorrichtung vorgesehen. Im erfindungsgemäßen Verfahren können diese wiederum getrennt als Alternative oder aber in beliebigen Kombinationen betrieben werden.
Bei Verwendung von z.B. Biomüll oder nachwachsenden Rohstoffen muss dieser vor Eintrag in den Vorversäuerungsreaktor zerkleinert werden, um die Desintegrationssysteme nicht zu beeinträchtigen und die hydrolytischen Vorgänge zu beschleunigen.

Wegen der säuretoleranten Eigenschaften der hydrolytischen und acidophilen Mikroorganismen kann die pH-Anpassung sowohl im Vorversäuerungsreaktor als auch im Zustrom zum Anaerobreaktor erfolgen. Diese pH-Adaption verringert in erheblichem Maße die Gefahr einer Übersäuerung im Anaerobreaktor und führt zu einer weiteren Stabilisierung des Gesamtprozesses.

### Effekte durch das neue Verfahren bzw. die neue Vorrichtung:

Durch die Dosierung von Stickstoff und Phosphor in den Vorversäuerungsreaktor werden die hydrolytischen und acidogenen Mikroorganismen optimal mit Nährstoffen versorgt. Eine Wachstumslimitierung dieser Organismen kann somit deutlich vermindert werden, was die mikrobiellen Vorgänge signifikant beschleunigt. Dies ermöglicht hohe Umsatzraten und kleine Reaktorvolumina für den Vorversäuerungsreaktor. Die Verweildauer des Substrats in der Vorversäuerungsvorrichtung bzw. dem Vorversäuerungsreaktor und die Vorversäuerung insgesamt kann von 6 - 24 h auf ca. 1 - 4 h verringert werden.

Eine solche Stickstoff und Phosphor -Dosierung erfolgt derzeit nicht in kommerziell erhältliche Reaktoren - dies geht deutlich über den derzeitigen Stand der Technik hinaus.

Der interne Schlammkreislauf durch z.B. Sedimentation der Mikroorganismen und gegebenenfalls Rückführung in den Vorversäuerungsreaktor führt zu höheren Biomassenkonzentrationen und einer Anreicherung von speziell angepassten Mikroorganismen im System. Dies beschleunigt zusätzlich die hydrolytischen und acidogenen Vorgänge, was zu geringen Reaktorvolumina und höheren Umsatzraten führt. Der Biomassenrückhalt kann z.B. durch Sedimentation im eigentlichen Reaktor erfolgen (Einbau von Schrägklärern vor dem Ablauf oder im ganzen Reaktor durch Ausschalten des Rührwerks in Analogie zu sequencing batch reactors - SBR-Verfahren) oder durch eine separate Sedimentationsstufe (in Analogie zu Belebungsverfahren) als auch durch interne und externe Filtration (in Analogie zu Membranverfahren in der Abwasserreinigung).

Interne Schlammkreisläufe für einen Vorversäuerungsreaktor existieren derzeit nach dem Stand der Technik nicht; auch eine Implementierung der Schlammrückführung geht deutlich über den derzeitigen Stand der Technik hinaus.

Die im Umlauf betriebenen Desintegrationsverfahren (z.B. Kavitationsverfahren, Elektroporation, etc.) unterstützen die Hydrolyse, insbesondere wenn organische Feststoffe zugeführt werden (Cosubstrate - z.B. Biomüll oder nachwachsende Rohstoffe).

Darüber hinaus unterstützen diese Desintegrationsverfahren die Freisetzung von Zellinhaltsstoffen bei z.B. der Schlammbehandlung (Schlammfaulung auf Kläranlagen). Damit wird ein hoher Aufschlussgrad unterschiedlicher Rohsubstrate erreicht und vor dem/für den nachfolgenden Anaerobreaktor (Acetogenese & Methanogenese - siehe Figur 1) relativ konstante Bedingungen eingestellt. Damit kann der eigentliche Anaerobreaktor deutlich unabhängiger von den eingesetzten Substraten betrieben werden, was zu einer signifikanten Steigerung der Betriebsstabilität führt.

Der durch die Desintegration herbeigeführte höhere Aufschlussgrad der Rohsubstrate (mikrobiell leicht verfügbar) ermöglicht anschließend im Anaerobreaktor höhere Mengen an erzeugtem Biogas oder anderen anaeroben Zwischenprodukten, was die Leistungsfähigkeit des Anaerobreaktors deutlich steigert - damit sind langfristig kleinere Anaerobreaktoren möglich.

Die im Bypass des Vorversäuerungsreaktors im Rücklaufstrom betriebenen Desintegrationsverfahren erlauben durch Änderung der Betriebszeit/Volumenströme und Variation der eingetragenen Leistung eine Anpassung des Aufschlussgrades in Abhängigkeit der eingesetzten Rohsubstrate. Damit kann unmittelbar auf die Qualität und Quantität der zuströmenden Rohsubstrate reagiert werden, um einen optimalen Aufschlussgrad für die nachfolgende Anaerobstufe zu erreichen.

Desintegrationsverfahren werden derzeit nach dem Stand der Technik nicht im Vorversäuerungsreaktor verwendet; eine Implementierung von Desintegrationsverfahren geht deutlich über den derzeitigen Stand der Technik hinaus.

### Anwendungspotential des neuen Verfahrens

Durch das vorgestellt Verfahren können nunmehr anaerobe Fermentationsanlagen nahezu unabhängig vom Substrat (z.B. nachwachsende Rohstoffe, Schlämme, organische Abfälle) betrieben werden, was den oben genannten Wirtschaftszweigen, insbesondere kleinen (landwirtschaftlichen) bis mittleren (kommunalen) Anlagen außerhalb der Großstädte, eine erweiterte Wachstumsperspektive bietet.

Die vorliegende Erfindung wird in den folgenden Figuren weiter verdeutlicht, ohne jedoch darauf beschränkt zu sein. Die zitierten Referenzen sind hiermit durch Bezugnahme vollständig aufgenommen.

### Figuren

- **Figur 1.**: Separation des anaeroben Abbaus durch Vorversäuerung.
- **Figur 2.**: Zweistufige Betriebsweise durch vorgeschaltete Vorversäuerung.
- **Figur 3.**: Verfahrensfließbild der erweiterten Vorversäuerungsstufe.

### Referenzen

[1] Bischofsberger, W.; Dichtl, N.; Rosenwinkel, K.-H.; Seyfried, C.F.; Böhnke, B.: Anaerobtechnik. 2. Auflage, Springer Verlag Berlin Heidelberg, 2005.
[2] H. Meyer: Leistungsfähigkeit anaerober Reaktoren zur Industrieabwasserreinigung, Veröffentlichungen des Instituts für Siedlungswasserwirtschaft und Abfalltechnik der Universität Hannover, Heft 128, 2004.
[3] Mitglieder der DWA-Arbeitsgruppe IG-5.1: Auswahl und Bewertung von Systemen und Reaktoren zur anaeroben Industrieabwasserbehandlung. Korrespondenz Abwasser, 59 (1), 36-44, 2012.
[4] Mitglieder der DWA-Arbeitsgruppe IG-5.1: Anaerobe Reaktoren und ihre Einsatzbereiche. Korrespondenz Abwasser, 56 (11), 1147 -1152, 2009.
[5] Weiland, P., Wulfert, K.: Fixed Bed Reactors for Anaerobic Treatment of High Strength Wastewaters - Development and Application, BTF - BIOTECH-FORUM, Heft 3, S. 151-158, 1986.
[6] Weiland, P.; Wulfert, K.: Entsorgung einer Bioethanol-Demonstrationsanlage, Chem. Ind., 97-99, 1986.
[7] Alexiou, I. E. Anderson, G. K., Evison, L. M.: Design of pre-acidification reactors for the anaerobic treatment of industrial wastewaters, Water Science & Technology, 29 (9), 199-204, 1994.
[8] Lettinga, G., Hulshoff Pol, L. W.: UASB-process design for various types of wastewaters, Water Science & Technology, 24 (8), 87-107, 1991.
[9] Moser, D.: Anaerobe Abwasserreinigung - Beeinflussende Faktoren der Versäuerung eines Zitronensäurefabrikabwassers, Wiener Mitteilungen Wasser - Abwasser - Gewässer, Band 173, 2002.
[10] Mitglieder der DWA-Arbeitsgruppe IG-5.1: Auswahl und Bewertung von Systemen und Reaktoren zur anaeroben Industrieabwasserbehandlung. Korrespondenz Abwasser, 59 (1), 36-44, 2012.

## Patentansprüche

1. Vorrichtung zur Vorversäuerung von organischem Substrat, insbesondere zur Verwendung im Rahmen eines nachgeschalteten anaeroben Abbauverfahrens, wobei die Vorrichtung umfasst:
(a) einen Reaktor ("Vorversäuerungsreaktor"), umfassend
(i) ein vorgeschaltetes Zerkleinerungsaggregat, für organisches Substrat und/oder Cosubstrat(e),
(ii) einen Zulauf für die Zuführung von Substrat und/oder Cosubstrat;
(iii) jeweils einen Abfluss und Zufluss zum Verbinden des Reaktors im Umlauf mit mindestens einem Desintegrations-System;
(iv) einen Ablauf, bevorzugt zum Verbinden des Reaktors mit einem anaeroben Reaktor;
(v) Rührmittel, wie Rührwerk oder einer internen oder externen Umwälzpumpe;
und wobei die Vorrichtung weiterhin umfasst:
(b) mindestens einen Desintegrations-System, das sich mit dem Reaktor (a) in Umlaufverbindung befindet; und/oder
(c) einen internen Schlammkreislauf für die Anreicherung von hydrolytischen und acidophilen Mikroorganismen;und/oder
(d) eine Zugabevorrichtung zur Dosierung von Phosphor und/oder Stickstoff in den Reaktor.

2. Vorrichtung gemäß Anspruch 1, umfassend zwei oder mehrere Desintegrations-Systeme (b),
und/oder wobei das oder die Desintegrations-Systeme (b) Mittel zur Durchführung eines Kavitationsverfahrens, Mittel zur Durchführung einer Elektroporation, eine Rührwerkskugelmühle, einen Scherspalthomogenisator, Mittel zur Durchführung eines Ultraschallverfahrens, Mittel zur Durchführung eines Elektroimpulsverfahrens, eine Lysat-Zentrifuge, Mittel zur Enzymzugabe, Mittel zur Durchführung von Autolyse, Mittel zur Durchführung von Nassoxidation, Mittel zur Ozondosierung, Mittel zur Durchführung von alkalischer Hydrolyse oder Mittel zur Durchführung von saurer Hydrolyse, oder Kombinationen davon umfasst (umfassen).

3. Vorrichtung nach einem der Ansprüche 1 oder 2, wobei das oder die Integrations-Systeme (b) mit dem Reaktor (a) im Bypass-Rücklaufstrom in Umlaufverbindung stehen.

4. Vorrichtung gemäß einem der Ansprüche 1 bis 3, wobei der interne Schlammkreislauf (c) umfasst:
Mittel zur Sedimentation im Reaktor, wie etwa einen Schrägklärer vor dem Ablauf, eine separate Sedimentationsstufe, und/oder Mittel zur internen und/oder externen Filtration.

5. Vorrichtung gemäß einem der Ansprüche 1 bis 4, weiterhin umfassend:
- mindestens ein Rückhaltesystem für die Anreicherung von hydrolytischen und/oder acidophilen Mikroorganismen und/oder für die Anreicherung von Feststoffen, zum Beispiel eine Abscheideeinrichtung oder Abscheidemittel zum Zurückhalten von Feststoffen und/oder von hydrolytischen und acidophilen Mikroorganismen, und/oder von Feststoffen, insbesondere ein (oder mehrere) Rückhaltesystem(e) ausgewählt aus:
Mitteln zur Filtration innerhalb oder außerhalb des Reaktors, insbesondere Mittel zur internen oder externen Crossflow-Filtration, beispielsweise Mikro- oder Ultrafiltration, wobei, bevorzugt, die Mittel zur Filtration in Nassaufstellung oder Trockenaufstellung vorgesehen sind; Mittel zur Feststoffrückhaltung, insbesondere mittels Zentrifugation; Mittel zur Sedimentation in einem dem Reaktor nachgeschalteten Becken, beispielsweise einem Zwischenklärbecken; Mittel zur Sedimentation im Reaktor, bevorzugt in einer oder mehreren strömungsberuhigten Zonen des Reaktors; und Kombinationen davon;
und/oder Mittel zur pH-Wert-Anpassung im Reaktor (a) oder im Ablauf des Reaktors.

6. Verfahren zur Vorbehandlung, insbesondere Vorversäuerung, von organischem Substrat, umfassend die Schritte:
- Bereitstellen eines zu behandelnden organischen Substrats in einer Vorversäuerungsvorrichtung umfassend einen Reaktor, insbesondere einer Vorrichtung gemäß einem der Ansprüche 1-5,
- Durchführen einer Hydrolyse und Acidogenese des organischen Substrats mit Hilfe von hydrolytischen und/oder acidophilen Mikroorganismen,
wobei im Rahmen der Hydrolyse/Acidogenese des organischen Substrats
(i) mindestens ein im Umlauf mit dem Reaktor verbundenes/betriebenes Desintegrations-Verfahren durchgeführt; und/oder
(ii) ein innerhalb des Reaktors befindlicher Schlammkreislauf für hydrolytische und/oder acidophile Mikroorganismen betrieben; und/oder
(iii) Stickstoff und/oder Phosphor, nach Bedarf der Mikroorganismen, in den Reaktor eindosiert; und/oder
(iv) optional, eine pH-Wert-Anpassung durchgeführt
wird.

7. Verfahren nach Anspruch 6, wobei zwei oder mehrere Desintegrations-Verfahren (i) durchgeführt werden, und/oder wobei das oder die Desintegrations-Verfahren (i) Kavitationsverfahren, Elektroporation, Mahlen mit einer Rührwerkskugelmühle, Homogenisieren mittels eines Scherspalthomogenisators, Ultraschallverfahren, Elektroimpulsverfahren, Zentrifugation mittels einer Lysatzentrifuge, Aufschluss mittels Enzymzugabe, Autolyse, Nassoxidation, Ozondosierung, alkalische Hydrolyse, saure Hydrolyse, und/oder Kombinationen davon umfasst (umfassen).

8. Verfahren nach Anspruch 6 oder 7, weiterhin umfassend eine Anreicherung der hydrolytischen und/oder acidophilen Mikroorganismen, bevorzugt durch Sedimentation und/oder Filtration, insbesondere während oder nach der Hydrolyse des biologischen Substrats.

9. Verfahren nach einem der Ansprüche 6 bis 8, weiterhin umfassend
eine Vorbehandlung des organischen Substrats und/oder von Co-Substraten, beispielsweise Biomüll oder nachwachsenden Rohstoffe,
bevorzugt umfassend eine mechanische Zerkleinerung des organischen Substrats und/oder der Co-Substrate.

10. Verfahren nach einem der Ansprüche 6 bis 9, weiterhin umfassend das Zurückhalten von Feststoffen, optional kombiniert mit einem Zurückhalten von hydrolytischen und/oder acidophilen Mikroorganismen zum Aufbau eines internen Schlammkreislaufs.

11. Verwendung der Vorrichtung nach einem der Ansprüche 1 bis 5 und/oder des Verfahrens nach einem der Ansprüche 6 bis 10,
- in der Biogasherstellung, insbesondere der landwirtschaftlichen Biogasherstellung,
- zur Klärschlammbehandlung,
- in der industriellen Abwasserreinigung,
- in der kommunalen Abwasserbehandlung,
- in der kommunalen Schlammbehandlung,
- zur Behandlung von organischen Abfällen,
- zur Behandlung von nachwachsenden Rohstoffen,
- zur Behandlung von Schlämmen und/oder Abwässern aus der Nahrungsmittelindustrie, chemischen Industrie, oder pharmazeutischen Industrie, und/oder
- zur kombinierten Behandlung oben genannter Einsatzstoffe, insbesondere von Klärschlamm, industriellem Abwasser, kommunalem Abwasser, kommunalem Schlamm, organischen Abfällen, nachwachsenden Rohstoffen, Schlämmen und/oder Abwässern aus der Nahrungsmittelindustrie, chemischen Industrie und/oder pharmazeutischen Industrie, als Co-Substratbeigaben.

12. Verfahren zum anaeroben Abbau von organischem Substrat, umfassend:
a) Durchführung eines Verfahrens zur Vorversäuerung gemäß einem der Ansprüche 6 bis 10, bevorzugt in einer Vorrichtung gemäß einem der Ansprüche 1 bis 5, mit einem organischen Substrat;
b) Anaerober Abbau des (der) aus (a) resultierenden Produkts (Produkte) des Vorversäuerungsverfahrens zu organischen Zwischen- und Endprodukten, wie z.B. Acetat, Formiat, Propionat, Butyrat, Valerianat, Ethanol, Propanol, Butanol, Wasserstoff, weiteren organischen Säuren oder Alkoholen, und/oder Methan, bevorzugt mittels Acetogenese und/oder Methanisierung,
wobei a) räumlich und/oder zeitlich getrennt von b) durchgeführt wird.
